# EUROPÄISCHE PATENTSCHRIFT

(11) **EP 1 896 375 B1**
(45) Veröffentlichungstag und Bekanntmachung des Hinweises auf die Patenterteilung: **13.08.2014**
(21) Anmeldenummer: 06743176.7
(22) Anmeldetag: 20.06.2006
(51) Int. Cl.: C04B 28/02, C04B 40/00, C04B 24/38

(54) **CELLULOSEETHERZUSAMMENSETZUNG FÜR DIE EXTRUSION ANORGANISCHER FORMKÖRPER**
CELLULOSE-ETHER COMPOSITION FOR EXTRUDING INORGANIC SHAPED BODIES
COMPOSITION D'ETHER DE CELLULOSE POUR L'EXTRUSION DE CORPS FAÇONNES INORGANIQUES

(30) Priorität: 30.06.2005 DE 102005030521
(43) Veröffentlichungstag der Anmeldung: 12.03.2008
(73) Patentinhaber: Dow Global Technologies LLC, Midland, MI 48674 (US)
(72) Erfinder: BAYER, Roland, 29664 Walsrode (DE); SCHLESIGER, Hartwig, 29664 Walsrode (DE); AURIEL, Daniel, 29683 Bad Fallingbostel (DE)
(74) Vertreter: f & e patent
(86) Internationale Anmeldenummer: PCT/EP2006/005881
(87) Internationale Veröffentlichungsnummer: WO 2007/003269

(56) Entgegenhaltungen:
- EP-A- 1 426 349
- EP-A- 1 506 973
- EP-A- 1 506 979
- WO-A-2005/080290
- JP-A- 8 225 355
- US-A1- 2005 016 422
- US-A1- 2005 241 540

## Beschreibung

Die vorliegende Erfindung betrifft Additive, die eine Celluloseetherzusammensetzung zum Einsatz als Wasserretentions-, Plastifizierungs- und Gleitmittel bei der Extrusion anorganischer Massen enthalten. Ferner betrifft sie die Verwendung dieser Celluloseetherzusammensetzung in Baustoffen und anderen Mischungen sowie ein Verfahren zur Extrusion anorganischer Massen unter Einsatz dieser Additive. Der Einsatz dieser Methylcellulosezusammensetzung führt beim Extrusionsprozess zu verbesserten Prozesseigenschaften, zu einer höheren Oberflächenqualität des extrudierten Körpers sowie zu verbesserten Produkteigenschaften des extrudierten Körpers.

Die Extrusion anorganischer Massen wird seit vielen Jahren technisch angewendet, sie ist ein Verfahren, pastöse anorganische Mischungen durch Pressen durch einen Düsenmund in beliebige Profile zu formen. Die auf diese Weise erhaltenen Elemente lassen sich vielfältig einsetzen, insbesondere in Bauanwendungen. Extrudierte anorganische Massen können unterschiedliche Zusammensetzungen haben, technisch und wirtschaftlich bedeutsam sind unter anderem extrudierte Zementmassen. Diese enthalten grundsätzlich Zement als Bindemittel, eventuell auch weitere Bindemittel, ferner Zuschläge (Sande) und/oder Leichtzuschläge, eventuell auch Fasern und/oder andere Additive sowie Celluloseether, insbesondere Methylcellulose als Wasserrückhalte-, Plastifizier- und Gleitmittel.

Unter Methylcellulose werden in dieser Schrift alle methylgruppenhaltigen Celluloseether wie Methylcellulose, Methylhydroxyethylcellulose, Methylhydroxypropylcellulose, Methylhydroxyethylhydroxypropylcellulose, Methylethylhydroxyethylcellulose, Methylhydroxyethylhydroxybutylcellulose sowie Celluloseether, die sowohl Methylgruppen als auch längerkettige hydrophobe Seitenketten enthalten, verstanden. Die Einsatzmenge der Methylcellulose liegt im Allgemeinen zwischen 0,1 und 6%.

Ob sich eine Masse unter Gesichtspunkten der Wirtschaftlichkeit extrudieren lässt und zu qualitativ hochwertigen Produkten mit guter Verkaufbarkeit verarbeiten lässt, wird durch verschiedene Parameter bestimmt:
1. Prozessparameter: Die Masse muss sich mit möglichst geringem Druck sowie möglichst hoher Geschwindigkeit homogen aus dem Düsenmund austragen lassen. Außerdem sollte sie nach Austritt aus dem Düsenmund die geringstmögliche Verformung (Aufweitung am Düsenmund, Breitenausdehnung) in Querrichtung zur Austragsrichtung aufweisen.
2. Produkteigenschaften: Die Oberfläche der extrudierten Masse spielt eine entscheidende Rolle. Sie sollte rissfrei und möglichst glatt sein. Gute Oberflächeneigenschaften sind vor allem bei höheren Extrusionstemperaturen ein Problem, die sich gewöhnlich nach längerer Extrusionsdauer einstellen. Erhöhte Festigkeiten oder ein schnelleres Abbinden der extru-dierten Produkte können weitere wünschenswerte Eigenschaften sein.

Dieser Erfindung liegt entsprechend die Aufgabe zugrunde, ein Wasserretentions-, Plastifizierungs- und Gleitmittel zu finden, welches verbesserte Eigenschaften hinsichtlich des Extrusionsprozesses, der Oberflächenqualität des Produktes und der physikalischen Produkteigenschaften aufweist.

WO 01/16048A1 (James Hardie) beschreibt eine Mischung von viskositätserhöhenden Mitteln (als solche werden unter anderem verschiedene Celluloseether genannt) und Dispergiermitteln (als solches werden sulfonierte Melamin-Formaldehyd-Harze als auch Polycarboxylate genannt, die üblicherweise als Fließmittel bezeichnet werden) als Additiv in extrudierten Zementmassen, mit dem Ziel, durch eine Synergie zwischen beiden Komponenten eine Einsparungsmöglichkeit an Celluloseether zu erreichen. Ferner wurden Vorteile wie bessere Oberfläche, geringerer Extrusionsdruck und teilweise erhöhte Extrusionsgeschwindigkeit angegeben. Aus WO 01/16048A1 ist dem Fachmann allerdings keineswegs ersichtlich, daß die nachfolgend beschriebene erfindungs-gemäße Abmischung aus drei Komponenten, nämlich aus Celluloseether, Fließmittel und Entschäumer in bestimmten Verhältnissen trotz eingestelltem (auf gleiche Konsistenz eingeregeltem) Wasserbedarf der extrudierten Masse die weiter unten genannten sechs Vorteile ergibt.

EP1266877A2 (Shin-Etsu) offenbart eine hydraulisch abbindende Mischung, die unter anderem einen Verdicker (Celluloseether) und einen Entschäumer (enthaltend Polyethergruppen oder -kom-ponenten) beinhaltet. Es wird beobachtet, dass die lineare Breitenaufweitung der ausgetragenen Masse am Düsenmund ("spring back") reduziert wird. Allerdings ergeben die Mischungen nach EP 1266877 A2 sehr steife Massen mit schlechten Extrusionseigenschaften.

In WO 03/024884 A1 (Shin-Etsu und NMB) werden Abmischungen aus Polycarboxylsäurecopolymer und/oder einem Salz daraus mit wasserlöslichen Celluloseethern und einem Entschäumer beschrieben. Das Mischungsverhältnis zwischen der ersten und zweiten Komponente, zwischen Polycarboxylsäurecopolymer oder Salzen davon und dem Celluloseether liegt bevorzugt bei 50:50 bis 99:1. Die hohe Lagerfähigkeit der flüssigen Mischung der drei Komponenten wird besonders hervorgehoben.

Es hat sich jedoch gezeigt, dass in der Extrusion zementgebundener Massen die in WO 03/024884 A 1 empfohlenen Verhältnisse der drei Komponenten untereinander sowie zum Bindemittel Zement ungeeignet sind.

Es wurde nun gefunden, dass bestimmte Mischungen der drei Komponenten Celluloseether, Polycarboxylatether-Verflüssiger und Entschäumer zueinander in einer anorganischen Mischung zu den folgenden Vorteilen führt, nämlich:
1. Zu niedrigerem Extrusionsdruck,
2. Zu höherer Extrusionsgeschwindigkeit,
3. Zu einer geringeren Aufweitung des extrudierten Profils nach Austritt aus dem Düsenmund ("lineare Breitenausdehnung" in %),
4. Zu einer stärkeren Kohäsion der extrudierten Masse, einhergehend mit größerer Duktilität und Plastizität der Masse,
5. Zu glatteren Oberflächen mit geringerer Rissbildung, und
6. zu höheren Druck- und Biegefestigkeiten.

Diese Vorteile werden beobachtet, wenn der Anteil der drei Komponenten Celluloseether (A), Fließmittel (B) und Entschäumer (C) in der Celluloseetherzusammensetzung 55-99,8 Gew.-% Celluloseether, 0,19-45 Gew.% Fließmittel und 0,01-25 Gew.% Entschäumer, bevorzugt 58-99,5 Gew.-% Celluloseether, 0,45-41.95 Gew.% Fließmittel und 0,05-22 Gew.% Entschäumer, ganz besonders bevorzugt 60-99 Gew.% Celluloseether, 0,9-39.9 Gew.% Fließmittel und 0,1-20 Gew.-% Entschäumer beträgt, wobei die Gewichtsprozente jeweils auf die Summe der Komponenten (A), (B) und (C) bezogen sind.

Gegenstand der Erfindung ist daher eine Celluloseetherzusammensetzung als Additiv zur Extrusion anorganischer Massen, enthaltend (A) 55-99,8 Gew.% Celluloseether, (B) 0,19-45 Gew.% Fließmittel und (C) 0,01-25 Gew.-% Entschäumer.

Der Einsatz dieser drei Komponenten in einem anderem als dem beanspruchten Verhältnis führt nicht zu den beschriebenen erfindungsgemäßen Resultaten.

Bevorzugt liegt das Mischungsverhältnis zwischen Fließmittel und Celluloseether bei 30:70 bis 1:99, besonders bevorzugt bei 35:65 bis 1,5: 98,5, ganz besonders bevorzugt bei 40:60 bis 2:98, wobei sich die angegebenen Verhältnisse auf Gewichtsanteile beziehen.

Bevorzugt beträgt der Anteil von Entschäumer, bezogen auf Celluloseether,: 0,05-20 Gew.-%, besonders bevorzugt 0,1- 15 Gew.%, ganz besonders bevorzugt 0,15-5 Gew.%.

Typischerweise wird die erfindungsgemäße Celluloseetherzusammensetzung in einer Menge von 0,1-6 Gew.% bezogen auf die anorganische Masse eingesetzt.

Sofern die anorganische Masse auf Zement basiert, beträgt der Anteil an Fließmittel üblicherweise 0,01 bis 4 Gew.%, bevorzugt 0,01 bis 3 Gew.%, besonders bevorzugt 0,015 bis 2 Gew.-% bezogen jeweils auf Zement. Der Anteil an Celluloseether beträgt üblicherweise 0,05 bis 10 Gew.%, bevorzugt 0,08 bis 7 Gew.%, besonders bevorzugt 0,1 bis 5 Gew.%, bezogen auf Zement.

Geeignete Celluloseether (A) sind insbesondere ionische Celluloseether wie Sulfoethylcellulose oder Carboxymethylcellulose und deren Salze, oder nichtionische Celluloseether, wie Alkyl-cellulosen, Hydroxyalkylalkylcellulosen oder Hydroxyalkylcellulosen, insbesondere Methylcellulose, Methylhydroxyethylcellulose, Methylhydroxypropylcellulose, Hydroxyethylcellulose, Ethylhydroxyethylcellulose, Methylethylhydroxyethylcellulose, Methylhydroxyethylhydroxypropyl-cellulose, Methylhydroxyethylhydroxybutylcellulose oder Cellulosether, die gleichzeitig Methyl-gruppen und längerkettige hydrophobe Seitenketten enthalten sowie Mischungen aus vorgenannten Produkten.

Die Viskositäten der oben genannten Celluloseether liegen in der Regel zwischen 400 und 200 000 mPas, gemessen in 2 %iger Lösung bei 20°C in einem Haake Rotationsviskosimeter.

Geeignete **Fließmittel** (B) sind beispielsweise Casein, Polycarboxylsäuren und deren Salze, Polymere, die sowohl Carboxylsäuren-Monomere oder deren Salze, als auch Carboxylatether-Monomere, Carboxylsäureester-Monomere, und andere Carboxylsäurederivate vernetzende Bisacrylate und ähnliche Monomere enthalten. Bevorzugt werden unter den Fließmitteln verstanden: Homo-, Co- und Terpolymere der Acryl-, Methacryl-, Croton-, Malein-, Fumarsäure und ähnlicher mono-und bifunktioneller Säuren sowie deren Salze, Ester und Ether verstanden. Unter die Ether fallen beispielsweise Polyalkylenglykolmono(meth)acrylate wie Triethylenglykolmonoacrylat und Polyethylenglykolmonoacrylat (mit Polyethylenglykolmolmasse von 200-2000 g/mol), aber auch ungesättigte Polyalkylenglykolether ohne Säuregruppe. Ganz besonders bevorzugt sind: Homo-, Co- und Terpolymere der Acryl- und Methacrylsäure, deren bifunktioneller Säuren sowie deren Salze, Ester und Ether. Unter die Ether fallen beispielsweise Polyalkylenglykolmono(meth)-acrylate wie Triethylenglykolmonoacrylat und Polyethylenglykolmonoacrylat (mit Polyethylenglykolmolmasse von 200-2000 g/mol), aber auch ungesättigte Polyalkylenglykolether ohne Säure-gruppe.

Unter Fließmitteln wird hier ausdrücklich nicht die Klasse der Melamin- oder Melamin-Formal-dehydsulfonate, der Naphthalinsulfonate, der Ligninsulfonate oder deren Abmischungen verstanden.

Besonders bevorzugte Fließmittel sind Polycarboxylsäurecopolymere und deren Salze.

Geeignete **Entschäumer** (C) sind insbesondere reine Substanzen oder Mischungen in flüssiger oder fester Form, die folgendes enthalten: Alkylenglykol-homo-, co-, ter- und blockcopolymere, beispielsweise auf Basis von Ethylen- oder Propylenoxid, Addukte von Alkylenoxiden, Alkylenglykolether von höheren Alkoholen, Fettsäureester, Alkylenglykolfettsäureester, Sorbitfettsäureester, Polyoxyalkylensorbitfettsäureester, Anlagerungsprodukte von Ethylenoxid und Propylenoxid and Acetylen, Phosphatester wie Tributylphosphat, Natriumoctylphosphat und ähnlichen sowie alle polyethergruppenhaltigen Verbindungen oder polyethergruppenhaltigen Abmischungen mit entschäumender Wirkung.

Besonders bevorzugt sind Alkylenglykol-homo-, co-, ter- und blockcopolymere, beispielsweise auf Basis von Ethylen- oder Propylenoxid, Addukte von Alkylenoxiden, Alkylenglykolether von höheren Alkoholen, Fettsäureester, Alkylenglykolfettsäureester und ähnlichen sowie alle polyethergruppenhaltigen Verbindungen oder polyethergruppenhaltigen Abmischungen mit entschäumender Wirkung.

Ganz besonders bevorzugt sind Alkylenglykol-homo-, co-, ter- und blockcopolymere, beispielsweise auf Basis von Ethylen- oder Propylenoxid, Addukte von Alkylenoxiden, Alkylenglykolether von höheren Alkoholen sowie alle polyethergruppenhaltigen Verbindungen oder polyethergruppenhaltigen Abmischungen mit entschäumender Wirkung.

Die Celluloseetherzusammensetzung kann neben den genannten Komponenten (A) bis (C) auch noch **weitere Additive** enthalten wie beispielsweise Hydrophobiermittel, Redispersionspulver, Superabsorber auf Basis von vernetzten Acrylaten und Polysacchariden, Gleitmittel (wie beispielsweise Polyethylenoxid- Homo-, Co-und Terpolymere), Tenside, Beschleuniger, Verzögerer, Fettsäuren und deren Ester, Polymere auf Basis von Säuren, Salzen, Amiden und Estern der Acrylsäuren und Methacrylsäuren, Polysaccharide wie natürliche oder derivatisierte Stärken, Xanthane, Glucane, Welan, Guar und verwandte Polysacchariden, Polyvinylalkohole einschließlich ihrer Derivate und Polymere auf Basis von Urethanen.

In einer typischen Anwendung wird die erfindungsgemäße Celluloseetherzusammensetzung einer Mischung anorganischer Komponenten, die aus 20-100 Teilen Bindemittel, 0-70 Teilen Zuschlägen, 0-30 Teilen Leichtzuschlägen, 0-20 Teilen Fasern und eventuell anderen Additiven besteht, in einer Menge von 0,1-3 Gew.% bezogen auf die Mischung anorganischer Komponenten zugesetzt.

Unter Bindemitteln werden hier alle anorganischen Bindemittel wie Zement, Gips, Kalkhydrat, Branntkalk, Ton/Lehm, Silikate, spezielle Flugaschen und keramische Bindemittel verstanden, bevorzugt aber alle Zement- und Gipsarten sowie dispersionsgebundene Bindemittel und keramische Massen.

Unter **Zuschlägen** werden hier alle Arten von Sanden und Steinmehlen verstanden, wie sie üblicherweise in Baumaterialien eingesetzt werden. Dies sind insbesondere Kiese, Brech- und Rundkornsande, Splitte, Aschen und Mehle auf Basis von Quarz, Kalk (Calciumcarbonat), Dolomit, Kaolin, Marmor, Glas, verschiedenen Arten von Bauschutt, Recyclingmaterial, spezielle Flugaschen, Tone, Bentonite und andere Schichtsilikate. Grundsätzlich lassen sich Zuschläge unterschiedlichster Korngrößen etrudieren, es ist bei der Zusammenstellung der Zuschläge entsprechend dem Anforderungsprofil möglich, bestimmte Kornfraktionen miteinander zu kombinieren, um bestimmte Eigenschaften optimal einzustellen.

**Leichtzuschläge** sind Zuschläge mit besonders niedriger Dichte. Diese können mineralischer Herkunft sein wie beispielsweise Perlite (Blähton), Blähglas, geblähte Calciumsilikate oder hochporöse natürliche Sande auf Quarz- oder Kalkbasis, sie können aber auch organischen Ursprungs sein wie expandiertes Polystyrol, Polyurothanschaumstoff, Kork usw..

Unter **Fasern** werden hier alle Arten von Natur- oder synthetischen Fasern verstanden wie beispielsweise Fasern auf Basis von Cellulose, Bambus, Kokos, Polyethylen, Polypropylen, Polyamid, Polyacrylnitril, Kohlenstoff, Glas, Keramik und andere mineralische Fasern. Deren Faserlängen und -dicken können über weite Bereiche variiert werden, um bestimmte Produkteigenschaften zu erzielen.

Die Zugabe der Celluloseetherabmischung (aus Celluloseether, Polycarboxylatether-Fließmittel und Entschäumer) muss nicht in einem separaten Schritt erfolgen, sondern es können auch die mindestens drei Einzelkomponenten einzeln mit den anderen Mörtelbestandteilen vermischt werden.

Ein weiterer Gegenstand der Erfindung ist ein Verfahren zur Extrusion anorganischer Massen unter Zumischung der vorstehend beschriebenen Celluloseetherzusammensetzung zur extrudierenden Masse. Erfindungsgemäßer Gegenstand ist dabei ein Verfahren zur Extrusion anorganischer Massen, dadurch gekennzeichnet, dass eine erfindungsgemäße Celluloseetherzusammensetzung zu 0,1-3 Gew.% einer Mischung anorganischer Komponenten, bestehend aus 20-100 Teilen Bindemittel, 0-70 Teilen Zuschlägen, 0-30 Teilen Leichtzuschlägen, 0-20 Teilen Fasern und eventuell anderen Additiven besteht, mit Wasser zugemischt wird und bis zum Erhalt zu einer homogenen Masse gemischt und/oder geknetet wird und diese Masse durch einen Düsenmund einer Strangpresse extrudiert wird.

Das erfindungsgemäße Verfahren wird durchgeführt, indem alle Rohstoffe in beliebiger Reihenfolge miteinander vermischt werden. Im allgemeinen werden alle Trockenkomponenten zunächst trocken vorgemischt, dann mit einer bestimmten Menge Wasser versetzt und erneut gemischt. Es ist jedoch auch möglich, die Trockenstoffe mit einer wässrigen Lösung der Celluloseetherabmischung zu versetzen (dies wird in der Regel jedoch zu einem schwer einzuarbeitenden Gel führen) oder sämtliche Komponenten und das Wasser gleichzeitig zu mischen. Es ist ebenfalls möglich, einen Teil oder alle Sande/Zuschlagsstoffe mit einer Feuchte von weniger als 10 % zuzumischen. Nachdem alle Komponenten miteinander vermischt worden sind, werden sie anschließend in einem Ein- oder Zweiwellenextruder verdichtet und durch ein Mundstück ausgepresst. Es ist möglich, Extuder mit und ohne Vakuumkammer und Extruder mit oder ohne Kühlung einzusetzen. Zwischen Mischen und Extrudieren kann auch noch ein Knetschritt in einem handelsüblichen Kneter eingeschaltet werden.

Ein weiterer Gegenstand der Erfindung ist ein Verfahren zur Herstellung einer Celluloseetherzusammensetzung, die als Additiv zur Extrusion mineralischer Massen eingesetzt wird, dadurch gekennzeichnet, dass man (A) 55-99,8 Gew.% Celluloseether, (B) 0,19-45 Gew.-% Fließmittel und (C) 0,01-25 Gew.% Entschäumer und gegebenenfalls weitere Additive im trockenen oder pastösen/gelartigen Zustand mischt.

Ein weiterer Gegenstand der Erfindung ist die Verwendung der erfindungsgemäßen Celluloseetherzusammensetzung als Additiv zur Herstellung zementgebundener Formkörper durch Extrusion.

### Beispiele

Die nachfolgenden Beispiele sollen die erfindungsgemäße Verwendung erläutern, ohne die Erfindung zu beschränken:

Durchführung des Abmischens und Extrudierens: 50 Teile Portlandzement CEM I 32,5R, 50 Teile Quarzmehl, 4,5 Teile Fasern und 1 Teil Celluloseetherzusammensetzung (alle Teile temperiert) werden zunächst trocken in einem Wirbelbettmischer homogen gemischt, anschließend wird temperiertes Wasser (Mengenangaben siehe unten) zugegeben, die Masse weitergemischt und in einem Kneter einige min geknetet. Anschließend wird die Masse sofort in den Zuführtrog des temperierten Einwellenextruders eingefüllt. Die Masse wird durch eine Lochplatte gepresst und zur Entgasung durch die Vakuumkammer geführt, durch eine Profildüse gepresst und auf ein Förderband ausgetragen. Alle erfindungsgemäß extrudierten Massen wurden hinsichtlich ihres Wasserbedarfs auf eine übliche Konsistenz der austretenden Masse eingestellt, die nicht erfindungsgemäßen Beispiele wurden alternativ auch auf den Wasserbedarf der Referenz eingestellt, wie es in den oben zitierten Patentanmeldungen WO 01/16048A1, EP1266877A2 und WO 03/024884 A1 durchgeführt wird.

### Beispiel 1 bis 10

### Besprechung der Versuchsresultate:

Beispiele 1, 2 und 3: Die Celluloseetherzusammensetzungen der Beispiele 2 und 3 zeigen gegenüber einem reinen Celluloseether (Beispiel 1, nicht erfindungsgemäß) eine erhebliche Verbesserung hinsichtlich Druckreduzierung, Geschwindigkeitserhöhung, Oberflächenqualität, Reduzierung der linearen Ausdehnung am Düsenmund, Kohäsion sowie Biege- und Druckfestigkeit, obwohl sie in WO 03/024884 A1 als ungeeignet beschrieben werden. Umgekehrt verhält es sich mit den Beispielen 5-8. Hier dient Beispiel 4 als Referenz zur Darstellung der Meßwerte eines reinen Celluloseethers und zur Wiederholung der Referenz. Es zeigt sich, dass Mischungen der drei Bestandteile in den Verhältnissen, wie sie in WO 03/024884 A1 empfohlen werden, in den Beispielen 5 und 7, wo die Konsistenz der Masse auf die der Referenz eingestellt wurde, zu einer erheblichen Verschlechterung der Oberfläche führen, die bereits ein Ausscheidungskriterium ist. Wird dagegen die Masse mit demselben Wasserbedarf wie die Referenz extrudiert (Beispiel 6 und 8), so ist die Masse so weich, dass eine Formstabilität des Extrudates nicht mehr gewährleistet ist, was wiederum ein Ausscheidungskriterium ist.

Versuche 9 bis 14 dienen dazu, die Eignung verschiedener Entschäumer in Abmischung mit Methylcellulose alleine (entsprechend EP 1266877 A2) zu prüfen. Versuch 9 und 10 (Entschäumer T): Bei einem W/F von 0,28 ergibt sich eine zu steife Masse und selbst wenn der W/F auf 0,30 (Versuch 10) erhöht wird, sinkt diese nur sehr geringfügig ab. Damit sind beide Massen ungeeignet. Der hohe Wasserbedarf von Versuch 10 lässt außerdem sehr niedrige Festigkeiten erwarten.

Versuch 11 und 12 (Entschäumer Rhoximat DF 770 DD): Bei einem (üblichen) W/F von 0,280 ist zu steif, wird jedoch der W/F auf 0,305 erhöht, um die Steifheit der Masse in den normalen Bereich abzusenken, ergeben sich zwar gute Prozessparameter (Druck, Geschwindigkeit, Oberfläche, Aufweitung am Düsenmund), jedoch verhindert der hohe Wasserbedarf akzeptable Festigkeiten.

Versuch 13 und 14 (Agitan P 803): Es werden analoge Beobachtungen wie bei den beiden vorangegangenen Versuchspaaren gemacht.

Versuch 15 und 16: Gegenüber der Referenz (mit 0,9% Methylcellulose) in Versuch 15 zeigt Versuch 16 trotz zusätzlichem Einsatz von Fließmittel auf Basis von Melaminsulfonat keinerlei nennenswerte Verbesserungen, bei gleichem Wasserbedarf in beiden Ansätzen zeigt sich sogar nichtmal eine weichere Masse in Versuch 16, ein Beleg für die geringe Wirksamkeit von Fließmitteln auf Basis von Melaminsulfonaten.

Versuch 17 und 18: Der Einsatz von Melflux 2651 F dagegen zeigt einen größeren Effekt der Wassereinsparung. Jedoch zeigt sich beim Einregeln des Wasserbedarfs auf die richtige Konsistenz ein deutlicher (nachteiliger) Druckanstieg und eine geringfügige Verschlechterung der Oberflächenqualität.

| **Beispiel Nr.** | **Celluloseetherzusammen-setzung (A:B:C (1) (V 1)** | **W/F (2)** | **Druck (bar) (3)** | **Geschw. (cm/min) (4)** | **Oberfläche (++ bis--)** | **Lineare Ausdehnung (%) (5)** | **Steifigkeit (zu steif/ gut/ zu weich (6)** | **Kohäsion der Masse (+0/-/--) (7)** | **Biegedruckfestigkeit (N/mm²) (8)** | **Druckfestigkeit (N/mm²), (8)** | **Erfindunggemäß hinsichtlich Komponentenverhältnis 1, 2, 3 oder 4 nach** WO 03/024884 A1 **oder dieser Anmeldung (7)** |
|---|---|---|---|---|---|---|---|---|---|---|---|
| 1 | 100:0:0 | 0,270 | 19-20 | 115 | + | 3,3 | Gut | 0 | 15,6 | 44,7 | Nicht erfindungsgemäß, Referenz |
| 2 | 80:18,4:1, 6 | 0,268 | 12-13 | 128 | ++ | 1,25 | Gut | + | 16,9 | 53,0 | Mischung erfindungsgemäß nach dieser Anm., als ungeeignet beschrieben nach WO 03/024884 A1 (wegen des V 4) |
| 3 | 67:28:5 | 0,265 | 10-11 | 132 | ++ | 0,8 | Gut | + | 16,2 | 50,9 | Mischung erfindungsgemäß nach dieser Anm., als ungeeignet beschrieben nach WO 03/024884 A1 (wegen des V 4) |
| 4 | 100:0:0 | 0,260 | 23-24 | 110 | + | 4,3 | Gut | 0 | n.g. | n.g. | Nicht erfindungsgemäß, Wiederholung Referenz |
| 5 | 20:79,4:0, 6 | 0,220 | 11-14 | 123 | - | 0,8 | Gut | - | n.g. | n.g. | Mischung als geeignet beschrieben nach WO 03/024884 A1, nicht erfindungsgemäß nach dieser Anmeldung |
| 6 | 20:79,4:0, 6 | 0,260 (wie Ref. Nr. 6) | 2-4 | 164 | - | n.g. | Zu weich | Nicht aussagefähig, weil zu weich | n.g. | n.g. | Mischung als geeignet beschrieben nach WO 03/024884 A1, nicht erfindungsgemäß nach dieser Anmeldung |
| 7 | 5:94,5:0,5 | 0,215 | 12-18 | 91 | - | 2,3 | Gut | -- | n.g. | n.g. | Mischung als geeignet beschrieben nach WO 03/024884 A1, nicht erfindungsgemäß nach dieser Anmeldung |
| 8 | 5:94,5:0,5 | 0,260 | 3 | n.s.b. | n.s.b. | n.s.b. | n.s.b. (erheblich zu weich) | Masse erheblich zu weich, nicht zu beurteilen. | n.g. | n.g. | Mischung als geeignet beschrieben nach WO 03/024884 A1, Masse nicht transportierbar, nicht erfindungsgemäß nach dieser Anmeldung |
| 9 | 90:0:10 | 0,280 | 22-24 | 113 | + | 2,3 | Zu steif | - | n.g. | n.g. | Nicht erfindungsgemäß nach dieser Anmeldung, erfindungsgemäß nach EP 1266877 A2 |
| 10 | 90:0:10 | 0,30 | 19-23 | 116 | + | 1,5 | Zu steif | - | n.g. | n.g. | Nicht erfindungsgemäß nach dieser Anmeldung, erfindungsgemäß nach EP 1266877 A2 |
| 11 | 90:0:10 | 0,28 | 20-22 | 121 | + | 1,8 | Zu steif | 0 | n.g | n.g. | Nicht erfindungsgemäß nach dieser Anmeldung, erfindungsgemäß nach EP 1266877 A2 |
| 12 | 90:0:10 | 0,305 | 12-13 | 135 | ++ | 1,0 | Gut | 0 | 13,1 | 37,1 | Nicht erfindungsgemäß nach dieser Anmeldung, erfindungsgemäß nach EP 1266877 A2 |
| 13 | 90:0:10 | 0,290 | 16-17 | 117 | ++ | 1,5 | Zu steif | 0 | n.g. | n.g. | Nicht erfindungsgemäß nach dieser Anmeldung, erfindungsgemäß nach EP 1266877 A2 |
| 14 | 90:0:10 | 0,305 | 12 | 125 | ++ | 1,5 | Gut | 0 | 13,9 | 38,8 | Nicht erfindungsgemäß nach dieser Anmeldung, erfindungsgemäß nach EP 1266877 A2 |
| 15 | 100:0:0, jedoch Einsatzmenge 0,9% | 0,275 | 19-20 | 109 | + | 4,5 | Gut | 0 | 13,8 | 40,3 | Nicht erfindungsgemäß nach dieser Anmeldung, erfindungsgemäß nach WO 01116048 A1 |
| 16 | 90:10:0 | 0,275 | 18-21 | 113 | Gering schlechter als + | 4,8 | Gut | 0 | 14,8 | 44,6 | Nicht erfindungsgemäß nach dieser Anmeldung, erfindungsgemäß nach WÖ 01/16048 A1 |
| 17 | 90:10:0 | 0,275 | 13-14 | 122 | n.s.b. (zu weich) | n.s.b | Zu weich | 0 | n.s.b. | n.s.b. | Nicht erfindungsgemäß nach dieser Anmeldung, erfindungsgemäß nach WO 01/16048 A1 |
| 18 | 90:10:0 | 0,265 | 20-21 | 107 | Schlechter als + | 4,5 | Gut | 0 | 15,6 | 47,3 | Nicht erfindungsgemäß nach dieser Anmeldung, erfindungsgemäß nach WO 01/16048 A1 |

### Anmerkungen:

1. A: Methylcellulose, B: Fließmittel, C: Entschäumer, angegeben sind Gewichtsanteile, die Einsatzmenge der Mischung der drei Komponenten beträgt 1% (außer bei Versuch 15), bezogen auf Trockenmasse der mineralischen Komponenten. Es wurden folgende Ausgangsstoffe eingesetzt:
   a) als Methylcellulose in allen Versuchen: Walocel M-20678 (Wolff Cellulosics GmbH, Deutschland), Viskosität bei 20°C, 2%ige wässrige Lösung, Haake-Rotationsviskosimeter, 75-85000 mPas laut Spezifikation;
   b) als Fließmittel in Versuch 2, 3, 5 bis 8, 17 und 18: Melflux 2651 F (Degussa, Deutschland), in Versuch 16 Melaminsulfonat,
   c) als Entschäumer in Versuch 9 und 10: Entschäumer T (=Tributylphosphat, Bayer AG, Deutschland), in Versuch 11 und 12: Rhoximat DF 770 DD (Rhodia), in Versuch 2, 3, 5-8, 13 und 14: Agitan P 803 (Münzing Chemie, Deutschland).
2. W/F meint den Wasser/Feststofffaktor. Die eingesetzte Menge Wasser wird nur auf die Sand und Zementmenge gerechnet, Fasern und Additive werden nicht berücksichtigt. Beispielsweise meint ein W/F von 0,29, dass auf 100 g Sand und Zement 29 g Wasser eingesetzt werden.
3. Druck meint den gemessenen Druck kurz vor dem Düsenmund. Der Wert ist gemittelt über mindestens sechs Messungen.
4. Autrittsgeschwindigkeit der extrudierten Masse aus dem Düsenmund.
5. Lineare Ausdehnung in der Breite, bezogen auf die Breite des Mundstücks (= 100%).
6. Die Steifigkeit der Masse wird an den frisch extrudierten Proben geprüft. Sie ist ein Maß für die Konsistenz der Probe. Wird eine Masse zu steif ausgetragen, so führt die höhere Reibung der Teilchen aneinander und an den Extruderwandungen zu einem höheren Stromverbrauch, zu einem erhöhtem Verschleiß und zur Aufheizung der Masse; wird die Masse zu weich ausgetragen, so ist sie forminstabil. Dies führt bei der Extrusion von Hohlprofilen zu einem Einbruch der Stege, grundsätzlich aber wegen des höheren W/F-Wertes zu geringeren Festigkeiten.
7. Die Kohäsion meint den inneren Zusammenhalt der frisch extrudierten Probe. Eine gute Kohäsion zeigt sich beispielsweise daran, dass das extrudierte Profil beim Biegen/Verdrehen keine oder weniger Risse als üblich zeigt und zum Auseinanderreißen der frisch extrudierten, und noch plastischen Probe (mit der Hand) ein viel größerer Kraftaufwand notwendig ist als bei nicht erfindungsgemäß extrudierten Proben.
8. Festigkeiten gemessen nach 28 d Lagerung unter folgenden Bedingungen: 2 d bei 23 +/-2 °C in verschweißten Polyethylentüten bei Raumtemperatur in einer Stahlpresse mit Höhe 40 mm, weitere 5 d unter denselben Bedingungen außerhalb der Presse, dann 21 d bei 23 +/-2 °C und 50 +/-5% rel. Feuchte an der Luft.
9. Verhältnis 1 (V 1) meint das Verhältnis Celluloseether: Polycarboxylatetherfließmittel (bezogen auf Pulver) : Entschäumer
   Verhältnis 2 (V 2) Polycarboxylatetherfließmittelin Gew.%, bezogen auf Zement.
   Verhältnis 3 (V 3) meint das Verhältnis Celluloseether in Gew.%, bezogen auf Zement.
   Verhältnis 4 (V 4) meint das Verhältnis zwischen Polycarboxylatetherfließmittel und Celluloseether zwischen 100:0 und 0:100.
   Verhältnis 5 (V5) meint das Verhältnis von Entschäumer in Gew.%, bezogen auf Celluloseether.
   Weitere Anmerkungen:
10. Abkürzungen: n.g.: nicht gemessen, n.s.b.= nicht sinnvoll bestimmbar
11. Die Temperatur der extrudierten Massen lag in allen Fällen zwischen 37 und 44°C.

## Patentansprüche

1. Celluloseetherzusammensetzung als Additiv zur Extrusion anorganischer Massen, enthaltend (A) 55-99,8 Gew.-% Celluloseether, (B) 0,19-45 Gew.-% Fließmittel und (C) 0,01-25 Gew.-% Entschäumer.

2. Celluloseetherzusammensetzung gemäß Anspruch 1, **dadurch gekennzeichnet, dass** der Celluloseether (A) mindestens einen ionischen Celluloseether aus der Gruppe bestehend aus Carboxymethylcellulose und Sulfoethylcellulosen sowie deren Salzen enthält.

3. Celluloseetherzusammensetzung gemäß Anspruch 1, **dadurch gekennzeichnet, dass** der Celluloseether (A) mindestens einen nichtionischen Celluloseether aus der Gruppe bestehend aus Methylcellulose, Methylhydroxyethylcellulose, Methylhydroxypropylcellulose, Hydroyethylcellulose, Hydroxypropylcellulose, Ethylhydroxyethylcellulose, Methylethylhydroxyethylcellulose, Methylhydroxyethylhydroxybutylcellulose sowie Celluloseether, die sowohl Methylgruppen als auch längerkettige hydrophobe Seitenketten enthalten, enthält.

4. Celluloseetherzusammensetzung gemäß einem der Ansprüche 1 bis 3,
**dadurch gekennzeichnet, dass** das Fließmittel ein synthetisches Polymer ist, enthaltend ungesättigte Monomere auf der Basis von Carboxylatethern und/oder Carboxylsäuren und/oder Carboxylsäuresalzen.

5. Celluloseetherzusammensetzung gemäß einem der Ansprüche 1 bis 4,
**dadurch gekennzeichnet, dass** der Entschäumer eine reine Substanz oder Mischung in flüssiger oder fester Form ist, der Alkylenglykol-homo-, co-, ter- und blockpolymer auf Basis von Ethylen- oder Propylenoxid enthält.

6. Celluloseetherzusammensetzung gemäß einem der Ansprüche 1 bis 5,
**dadurch gekennzeichnet, dass** die Celluloseetherzusammensetzung pulverförmig ist.

7. Verfahren zur Extrusion anorganischer Massen, **dadurch gekennzeichnet, dass** eine Celluloseetherzusammensetzung gemäß einem der Ansprüche 1 bis 6 einer Mischung anorganischer Komponenten, bestehend aus 20 bis 100 Teilen Bindemittel, 0 bis 70 Teilen Zuschlägen, 0 bis 30 Teilen Leichtzuschlägen, 0 bis 20 Teilen Fasern und eventuell anderen Additiven, in einer Menge von 0,1 bis 3 Gew.-%, bezogen auf die Mischung anorganischer Komponenten, zugegeben wird, mit Wasser gemischt wird und bis zum Erhalt einer homogenen Masse gemischt und/oder geknetet wird und die Masse durch einen Düsenmund einer Strangpresse/eines Extruders extrudiert wird.

8. Verfahren zur Herstellung einer Celluloseetherzusammensetzung gemäß einem der Ansprüche 1 bis 6 **dadurch gekennzeichnet, dass** man (A) 55-99,8 Gew.-% Celluloseether, (B) 0,19-45 Gew.-% Fließmittel und (C) 0,01-25 Gew.-% Entschäumer und gegebenenfalls weitere Additive im trockenen, flüssigen oder pastösen/gelartigem Zustand mischt oder verknetet.

9. Verwendung einer Celluloseetherzusammensetzung gemäß einem der Ansprüche 1 bis 6 als Additiv zur Herstellung zementgebundener Formköper durch Extrusion.

10. Mischung anorganischer Komponenten bestehend aus 20 bis 100 Teilen Bindemittel, 0 bis 70 Teilen Zuschlägen, 0 bis 30 Teilen Leichtzuschlägen, 0 bis 20 Teilen Fasern und eventuell andere Additive sowie 0,1 bis 3 Gew.-% (bezogen auf die Mischung anorganischer Komponenten) einer Celluloseetherzusammensetzung gemäß einem der Ansprüche 1 bis 6.

11. Mischung anorganischer Komponenten gemäß Anspruch 10, **dadurch gekennzeichnet, dass** das Bindemittel Zement ist und der Anteil an Fließmittel 0,01 bis 4 Gew.-% und der Anteil an Celluloseether 0,05 bis 10 Gew.-% beträgt, bezogen jeweils auf den Zement.

## Claims

1. A cellulose ether composition as additive in the extrusion of inorganic compositions comprising (A) 55 - 99.8 % by weight of a cellulose ether, (B) 0.19 - 45 % by weight of a superplasticizer and (C) 0.01 - 25 % by weight of a defoamer.

2. The cellulose ether composition according to claim 1, **characterized in that** the cellulose ether (A) contains at least one ionic cellulose ether selected from the group consisting of carboxymethylcellulose and sulphoethylcelluloses and salts of each.

3. The cellulose ether composition according to claim 1, **characterized in that** the cellulose ether (A) contains at least one non-ionic cellulose ether selected from the group consisting of methyl cellulose, methylhydroxyethylcellulose, methylhydroxypropylcellulose, hydroethylcellulose, hydroxypropylcellulose, ethylhydroxyethylcellulose, methylethylhydroxyethylcellulose, methylhydroxyethylhydroxybutylcellulose and celluloseethers which contain methyl groups and longer-chain hydrophobic side chains.

4. The cellulose ether composition according to any of claims 1 to 3, **characterized in that** the superplasticizer is a synthetic polymer comprising unsaturated monomers on the basis of carboxylate ethers and/or carboxylic acids and/or salts of carboxylic acid.

5. The cellulose ether composition according to any of claims 1 to 4, **characterized in that** the defoamer is a pure substance or mixture in liquid or solid form containing an alkylene glycol homo-, co-, ter- and block polymer based on ethylene or propylene oxide.

6. The cellulose ether composition according to any of claims 1 to 5, **characterized in that** the cellulose ether composition is in powder form.

7. A method for extruding inorganic materials, **characterized in that** a cellulose ether composition according to any of claims 1 to 6 is added to a mixture of inorganic components comprising 20 to 100 parts of binder, 0 to 70 parts of aggregates, 0 to 30 parts of lightweight aggregates, 0 to 20 parts of fibers and optional other additives in an amount of 0.1 to 3 wt.-%, based on the mixture of inorganic components, is mixed with water and mixed and/or kneaded until a homogeneous composition is obtained, and extruding the homogeneous composition through a die of an extruder.

8. Method for the preparation of a cellulose ether composition according to any of claims 1 to 6, **characterized in that** (A) 55-99.8 wt.-% of cellulose ether, (B) 0.19-45 wt.-% of superplasticizer and (C) 0.01-25 wt.-% of defoamer and optionally further additives are mixed or kneaded in the dry, liquid or pasty/gel-like state.

9. Use of a cellulose ether composition according to any of claims 1 to 6 as additive for the production of cement-bound shaped bodies by extrusion.

10. Mixture of inorganic components consisting of 20 to 100 parts binder, 0 to 70 parts of aggregates, 0 to 30 parts of lightweight aggregates, 0 to 20 parts of fibers and optional additives, and 0.1 to 3 wt.-% based on the mixture of inorganic components of a cellulose ether composition according to any of claims 1 to 6.

11. Mixture of inorganic components according to claim 10, **characterized in that** the binder is cement, the amount of superplasticizer is 0.01 to 4 wt.-% and the amount of cellulose ether is 0.05 to 10 Gew.-%, in each case based on the cement.

## Revendications

1. Composition d'éther de cellulose en tant qu'additif pour l'extrusion de masses inorganiques, comprenant (A) 55-99,8 % en poids d'éther de cellulose, (B) 0,19-45 % en poids de fluidifiant et (C) 0,01-25 % en poids d'antimoussant.

2. Composition d'éther de cellulose conformément à la revendication 1, **caractérisée en ce que** l'éther de cellulose (A) comprend au moins un éther de cellulose ionique du groupe constitué de carboxyméthylcellulose et de sulfoéthylcelluloses ainsi que de leurs sels.

3. Composition d'éther de cellulose conformément à la revendication 1, **caractérisée en ce que** l'éther de cellulose (A) comprend au moins un éther de cellulose non ionique du groupe constitué de méthylcellulose, méthylhydroxyéthylcellulose, méthylhydroxypropylcellulose, hydroyéthylcellulose, hydroxypropylcellulose, éthylhydroxyéthylcellulose, méthyléthylhydroxyéthylcellulose, méthylhydroxyéthylhydroxybutylcellulose ainsi que d'éther de cellulose, lesquelles comprennent aussi bien des groupes méthyle que des chaînes latérales hydrophobes longues.

4. Composition d'éther de cellulose conformément à l'une des revendications 1 à 3, **caractérisée en ce que** le fluidifiant est un polymère synthétique comprenant des monomères insaturés à base d'éthers de carboxylate et/ou d'acides carboxyliques et/ou de sels d'acide carboxylique.

5. Composition d'éther de cellulose conformément à l'une des revendications 1 à 4, **caractérisée en ce que** l'antimoussant est une substance pure ou un mélange sous forme liquide ou solide qui comprend un un homo-, co-, ter- et copolymère bloc d'alkylèneglycol à base d'oxyde d'éthylène ou de propylène.

6. Composition d'éther de cellulose conformément à l'une des revendications 1 à 5, **caractérisée en ce que** la composition d'éther de cellulose est sous forme de poudre.

7. Procédé d'extrusion de masses inorganiques, **caractérisé en ce qu'**une composition d'éther de cellulose conformément à l'une des revendications 1 à 6 est ajoutée à un mélange de composants inorganiques, lequel est constitué de 20 à 100 parties de liant, de 0 à 70 parties de granulats, de 0 à 30 parties de granulats légers, de 0 à 20 parties de fibres et éventuellement d'autres additifs, dans une quantité de 0,1 à 3 % en poids par rapport au mélange de composants inorganiques, ladite composition étant mélangée avec de l'eau puis mélangée et/ou pétrie jusqu'à obtention d'une masse homogène, puis la masse est extrudée par une embouchure de buse d'une presse d'extrusion/d'une extrudeuse.

8. Procédé de fabrication d'une composition d'éther de cellulose conformément à l'une des revendications 1 à 6, **caractérisé en ce que** l'on mélange ou pétrit (A) 55-99,8 % en poids d'éther de cellulose, (B) 0,19-45 % en poids de fluidifiant et (C) 0,01-25 % en poids d'antimoussant et le cas échéant d'autres additifs à l'état sec, liquide ou pâteux/ayant la consistance d'un gel.

9. Utilisation d'une composition d'éther de cellulose conformément à l'une des revendications 1 à 6 en tant qu'additif pour la fabrication par extrusion de corps façonnés liés au ciment.

10. Mélange de composants inorganiques constitué de 20 à 100 parties de liant, de 0 à 70 parties de granulats, de 0 à 30 parties de granulats légers, de 0 à 20 parties de fibres et éventuellement d'autres additifs ainsi que de 0,1 à 3 % en poids (par rapport au mélange de composants inorganiques) d'une composition d'éther de cellulose conformément à l'une des revendications 1 à 6.

11. Mélange de composants inorganiques conformément à la revendication 10, **caractérisé en ce que** le liant est du ciment et que, respectivement par rapport au ciment, la part de fluidifiant est de 0,01 à 4 % en poids et la part d'éther de cellulose de 0,05 à 10 % en poids.
